# EUROPÄISCHE PATENTSCHRIFT

(11) **EP 3 746 830 B1**
(45) Veröffentlichungstag und Bekanntmachung des Hinweises auf die Patenterteilung: **31.07.2024**
(21) Anmeldenummer: 19702368.2
(22) Anmeldetag: 24.01.2019
(51) Int. Cl.: G02B 23/24, A61B 1/00, A61B 1/05, G02B 27/00

(54) **OPTISCHES SYSTEM EINES STEREO-VIDEOENDOSKOPS**
OPTICAL SYSTEM OF A STEREO-VIDEO ENDOSCOPE
SYSTÈME OPTIQUE D'UN STÉRÉO-VIDÉO-ENDOSCOPE

(30) Priorität: 01.02.2018 DE 102018102268
(43) Veröffentlichungstag der Anmeldung: 09.12.2020
(60) Teilanmeldung: 24176244.2
(73) Patentinhaber: OLYMPUS Winter & Ibe GmbH, 22045 Hamburg (DE)
(72) Erfinder: ZHAO, Jianxin, 22399 Hamburg (DE)
(74) Vertreter: Seemann & Partner Patentanwälte mbB
(86) Internationale Anmeldenummer: PCT/EP2019/051700
(87) Internationale Veröffentlichungsnummer: WO 2019/149609

(56) Entgegenhaltungen:
- US-A- 5 689 365
- US-A1- 2009 296 235
- US-A1- 2016 370 580
- US-A1- 2017 235 121

## Beschreibung

Die Erfindung betrifft ein optisches System eines Stereo-Videoendoskops mit seitlicher Blickrichtung, umfassend eine seitwärts blickende distale optische Baugruppe und eine proximale optische Baugruppe, wobei die distale optische Baugruppe in einer Lichteinfallsrichtung aufeinanderfolgend eine Eintrittslinse, eine als Prismeneinheit ausgebildete Ablenkeinheit und eine Austrittslinse auf einer gemeinsamen optischen Achse umfasst, und wobei die proximale optische Baugruppe einen linken und einen rechten Linsensystemkanal umfasst, wobei die Linsensystemkanäle gleichartig aufgebaut und parallel zueinander angeordnet sind und jeweils eine eigene optische Achse aufweisen.

Ferner betrifft die Erfindung ein Stereo-Videoendoskop mit seitlicher, insbesondere fester, Blickrichtung sowie eine Verwendung einer Linse für ein optisches System eines Stereo-Videoendoskops. Videoendoskope, bei denen das an einer distalen Spitze eines Endoskopschafts eintretende Licht durch ein optisches System auf einen oder mehrere Bildsensoren gelenkt wird, sind in verschiedenen Ausführungen bekannt. Es gibt Endoskope mit Geradeausblick, einer sog. 0°-Blickrichtung, Endoskope mit (fester) seitlicher Blickrichtung sowie Endoskope mit verstellbarer Blickrichtung (auch als V-DOV-Endoskope bezeichnet).

Außerdem sind Stereo-Videoendoskope bekannt, die dazu eingerichtet sind, ein stereoskopisches Bildpaar und/oder zwei stereoskopische Videokanäle aufzunehmen. Mit solchen Instrumenten ist es möglich, ein 3D-Abbild eines distal vor dem Ende des Endoskopschafts liegenden Objekts in einem Untersuchungs- oder Operationsraum zu erzeugen.

Stereo-Videoendoskope mit seitlicher Blickrichtung sind seitwärts blickende Endoskope mit einem festen vom Geradeausblick abweichenden Blickwinkel. Solche Endoskope umfassen vielfach eine Prismenanordnung aus mehreren Prismen, die die aus dem Objektraum unter einem Winkel zur Längsachse des Endoskopschafts in das optische System eintretenden Lichtstrahlen zweimal reflektieren und seitenrichtig in Richtung des Endoskopschafts umlenken. Ein solches Endoskop ist beispielsweise aus der DE 10 2014 206 513 A1 der Olympus Winter & Ibe GmbH, Hamburg, bekannt.

Eine Umlenkprismenanordnung eines solchen Stereo-Videoendoskops umfasst typischerweise zwei oder drei Prismen. Die Prismen sind an ihren gemeinsamen Grenzflächen vielfach miteinander verkittet. Bei einer solchen Umlenkprismenanordnung findet die Reflektion der einfallenden Lichtbündel an zwei sowohl zur optischen Achse der Eintrittslinse als auch zur Längsachse des Endoskopschafts schrägstehenden reflektierenden Grenzflächen eines zweiten oder dritten Prismas statt. Das zweite oder dritte Prisma der Umlenkprismenanordnung befindet sich in Lichteinfallsrichtung hinter einem ersten oder zweiten Prisma, welches hinter der Eintrittslinse angeordnet ist. Die schrägstehende reflektierende Grenzfläche des zweiten oder dritten Prismas, an der die zweite Reflexion stattfindet, bildet teilweise eine gemeinsame Grenzfläche mit dem vorderen Prisma, welches die einfallenden Lichtstrahlen zuerst durchlaufen.

Die Eintrittslinse des optischen Systems eines solchen Stereo-Videoendoskops definiert die optische Achse des optischen Systems. Das optische System umfasst Blenden oder Menisken, die ein Blickfeld bzw. die Öffnungswinkel der Optik festlegen. Innerhalb des Blickfeldes in das optische System einfallende Lichtbündel werden von dem optischen System auf einen oder mehrere Bildsensoren abgebildet. Lichtbündel, die von außerhalb des Blickfeldes oder durch Mehrfachreflexionen in das optische System einfallen, erzeugen so sogenannte "Geisterbilder" oder "Flares".

Eine bekannte Umlenkprismengruppe, in der solche Geisterbilder entstehen können, umfasst ein erstes Prisma und ein zweites Prisma, die miteinander verkittet sind. Das erste Prisma weist eine Eintrittsseite und eine Austrittsseite auf, wobei die Eintrittsseite gegenüber der Austrittsseite geneigt ist. Die Austrittsseite des ersten Prismas grenzt unmittelbar an eine zweite Eintrittsseite des zweiten Prismas an. Beispielsweise sind das erste und das zweite Prisma an diesen beiden Seiten miteinander verkittet. Das zweite Prisma umfasst ferner eine Reflexionsseite und eine zweite Austrittsseite. Licht, welches aus dem Bildfeld in die Umlenkprismengruppe einfällt, durchquert die Eintrittsseite des ersten Prismas und tritt an dessen Austrittsseite wieder aus. Das Licht gelangt anschließend unmittelbar durch die zweite Eintrittsseite in das zweite Prisma, wird an der Reflexionsseite innerhalb des zweiten Prismas reflektiert und verlässt dieses an der Austrittsseite.

US 5,689,365 A1 offenbart ein Stereo-Videoendoskop, bei dem eine distale optische Baugruppe mit einer seitwärtigen Blickrichtung vorgesehen ist. Bezogen auf die Lichteinfallsrichtung ist hinter der distalen optischen Baugruppe eine proximale optische Baugruppe mit zwei Linsensystemkanälen angeordnet.

Außerdem offenbart US 2009/0296235 A1 ein optisches System für Endoskope, das distalseitig im Endoskop angeordnet ist. Das optische System weist hierbei in Reihenfolge der Bildseite eine Einzellinse mit einer negativen Brechkraft, eine zementierte Linse mit positiver Brechkraft und eine Bildsensoreinheit auf.

Darüber hinaus zeigt US 2016/0370580 A1 eine Herstellung eines optischen Elements, wobei auf eine Oberfläche eines optischen Körpers mittels eines Tintenstrahldruckverfahrens Tinte mit einem lichtblockierenden Material aufgebracht wird.

US 2017/0235121 A1 offenbart ein optisches Endoskop-Objektivsystem, wobei im Fall eines engen Abstands zwischen einer vorderen und einer hinteren optischen Gruppe eine dazwischenliegende Aperturblende an einer Linsenoberfläche angebracht wird.

Ausgehend von diesem Stand der Technik besteht die Aufgabe der Erfindung darin, bei einem Stereo-Videoendoskop auf einfache Weise die Bildung von Geisterbildern zu verhindern.

Gelöst wird diese Aufgabe durch ein optisches System eines Stereo-Videoendoskops mit seitlicher Blickrichtung, umfassend eine seitwärts blickende distale optische Baugruppe und eine proximale optische Baugruppe, wobei die distale optische Baugruppe in einer proximale optische Baugruppe einen linken und einen rechten Linsensystemkanal umfasst, wobei die Linsensystemkanäle gleichartig aufgebaut und parallel zueinander angeordnet sind und jeweils eine eigene optische Achse aufweisen, das dadurch weitergebildet wird, dass die Austrittslinse im mittleren Bereich der Austrittslinse auf ihrer der Ablenkeinheit zugewandten Lichteintrittsseite mit einer lichtundurchlässigen Beschichtung und/oder auf ihrer der Ablenkeinheit abgewandten Lichtaustrittsseite mit einer lichtundurchlässigen Beschichtung ausgebildet ist.

Die Erfindung beruht auf dem Gedanken, dass die Austrittslinse der distalen optischen Baugruppe, die vorzugsweise an der Prismeneinheit der distalen optischen Baugruppe angeordnet ist, mit einer Beschichtung versehen ist, wodurch kein Licht im mittleren Bereich durch die Austrittslinse hindurchtritt und die Bildung von Geisterbildern in den Linsensystemkanälen minimiert oder verhindert wird.

Die Austrittslinse ist vorzugsweise als Prismalinse ausgebildet, wobei die Lichtstrahlen für den linken und rechten optischen Kanal Lichteinfallsrichtung aufeinanderfolgend eine Eintrittslinse, eine als Prismeneinheit ausgebildete Ablenkeinheit und eine Austrittslinse auf einer gemeinsamen optischen Achse umfasst, und wobei die proximale optische Baugruppe einen linken und einen rechten Linsensystemkanal umfasst, wobei die Linsensystemkanäle gleichartig aufgebaut und parallel zueinander angeordnet sind und jeweils eine eigene optische Achse aufweisen, das dadurch weitergebildet wird, dass die Austrittslinse auf ihrer der Ablenkeinheit abgewandten Lichtaustrittsseite mit einer lichtundurchlässigen Beschichtung ausgebildet ist.

Die Erfindung beruht auf dem Gedanken, dass die Austrittslinse der distalen optischen Baugruppe, die vorzugsweise an der Prismeneinheit der distalen optischen Baugruppe angeordnet ist, mit einer Beschichtung versehen ist, wodurch kein Licht im mittleren Bereich durch die Austrittslinse hindurchtritt und die Bildung von Geisterbildern in den Linsensystemkanälen minimiert oder verhindert wird.

Die Austrittslinse ist vorzugsweise als Prismalinse ausgebildet, wobei die Lichtstrahlen für den linken und rechten optischen Kanal zwischen dem Bereich der lichtdurchlässigen Beschichtung auf der Austrittslinse und dem äußeren Rand der Austrittslinse hindurchgelassen werden. Im mittleren Bereich der Austrittslinse, in dem die Beschichtung oder Beschichtungen auf der Austrittslinse aufgetragen sind, werden keine Lichtstrahlen hindurchgelassen. Durch die Beschichtung oder Beschichtungen weist die Austrittslinse einen ringförmigen oder ringartigen optischen Durchlassbereich auf.

Vorzugsweise ist die Austrittslinse im mittleren Bereich der Austrittslinse auf ihrer der Ablenkeinheit zugewandten Lichteintrittsseite mit einer lichtundurchlässigen Beschichtung ausgebildet.

In einer Weiterbildung des optischen Systems ist vorgesehen, dass die Beschichtung auf der Austrittslinse auf ihrer der Ablenkeinheit abgewandten Lichtaustrittsseite kreisförmig oder kreisartig ausgebildet ist und/oder dass die Beschichtung auf der Austrittslinse auf ihrer der Ablenkeinheit zugewandten Lichtaustrittsseite kreisförmig oder kreisartig ausgebildet ist.

Vorzugsweise ist die Austrittslinse für den linken Linsensystemkanal und den rechten Linsensystemkanal der proximalen optischen Baugruppe lichtdurchlässig, wobei insbesondere der optische Durchlassbereich der Austrittslinse ringartig oder ringförmig außerhalb der Beschichtung(en) ausgebildet ist.

Ferner ist es bevorzugt, dass die von der Ablenkeinheit abgewandte Seite der Austrittslinse konvex ist.

Vorzugsweise ist die Austrittslinse als konkav-konvexe Linse ausgebildet.

Des Weiteren ist bei dem optischen System bevorzugt, dass die Beschichtung auf der Austrittslinse auf ihrer der Ablenkeinheit abgewandten Lichtaustrittsseite als Antireflexbeschichtung ausgebildet ist und/oder dass die Beschichtung auf der Austrittslinse auf ihrer der Ablenkeinheit zugewandten Lichtaustrittsseite als Antireflexbeschichtung ausgebildet ist. Als Antireflexbeschichtung ist eine Beschichtung vorgesehen, um die Entstehung von Flares oder Geisterbilder zu verringern oder zu vermeiden.

Ferner ist es bevorzugt, dass die lichtundurchlässige Beschichtung oder die Antireflexbeschichtung als Chrombeschichtung ausgebildet ist.

Außerdem wird die Aufgabe gelöst durch ein Stereo-Videoendoskop mit fester, vorzugsweise seitlicher, Blickrichtung mit einem optischen System wie voranstehend beschrieben. Hierbei umfasst das optische System eine oder mehrere der zuvor genannten Ausführungsformen.

Des Weiteren wird die Aufgabe gelöst durch eine Verwendung einer Linse für ein optisches System eines Stereo-Videoendoskops mit fester Blickrichtung, wobei das optische System gemäß der voranstehend genannten Ausführungsformen ausgebildet ist, wobei die Linse als Austrittslinse einer distalen optischen Baugruppe ausgebildet ist und im mittleren Bereich der Austrittslinse auf ihrer einer Ablenkeinheit der distalen optischen Baugruppe abgewandten Lichtaustrittsseite mit einer lichtundurchlässigen Beschichtung ausgebildet ist.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch ein Stereo-Videoendoskop in vereinfachter perspektivischer Darstellung,
- Fig. 2: ein optisches System eines Stereo-Videoendoskops gemäß dem Stand der Technik in einer vereinfachten schematischen Schnittansicht,
- Fig. 3a: schematisch einen Querschnitt durch eine erfindungsgemäße Austrittslinse einer distalen optischen Baugruppe des Stereo-Videoendoskops und
- Fig. 3b: schematisch eine Ansicht der Austrittslinse der distalen optischen Baugruppe.

In den Zeichnungen sind jeweils gleiche oder gleichartige Elemente und/oder Teile mit denselben Bezugsziffern versehen, so dass von einer erneuten Vorstellung jeweils abgesehen wird.

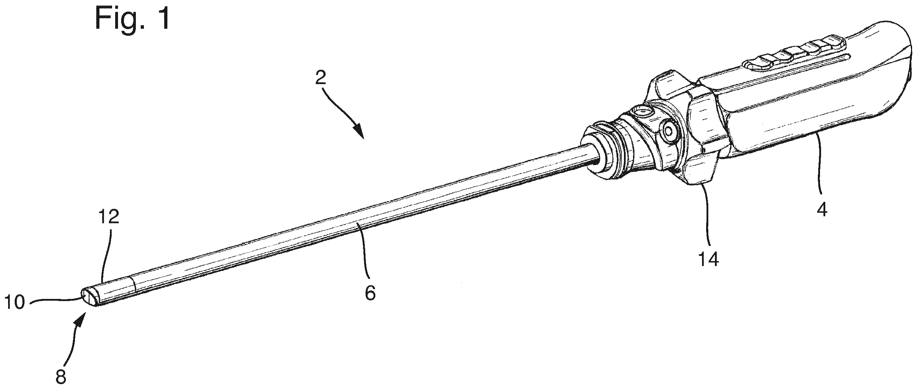

Fig. 1 zeigt in vereinfachter perspektivischer Darstellung ein bekanntes Endoskop 2 mit einem proximalen Handgriff 4 und einem starren Endoskopschaft 6. An einer distalen Spitze 8 des Endoskopschafts 6 befindet sich ein Sichtfenster 10. Daran schließt sich ein distaler Abschnitt 12 des Endoskopschafts 6 an. Im distalen Abschnitt 12 ist ein in Fig. 1 nicht sichtbares optisches System angeordnet, mit dem ein vor der distalen Spitze 8 des Endoskops 2 liegender Untersuchungs- oder Operationsbereich auf ebenfalls nicht dargestellte Bildsensoren abgebildet wird. An den Handgriff 4 schließt sich in distaler Richtung ein Drehrad 14 an, mit dem das im Inneren des Endoskopschafts 6 liegende optischen System azimutal verdreht werden kann.

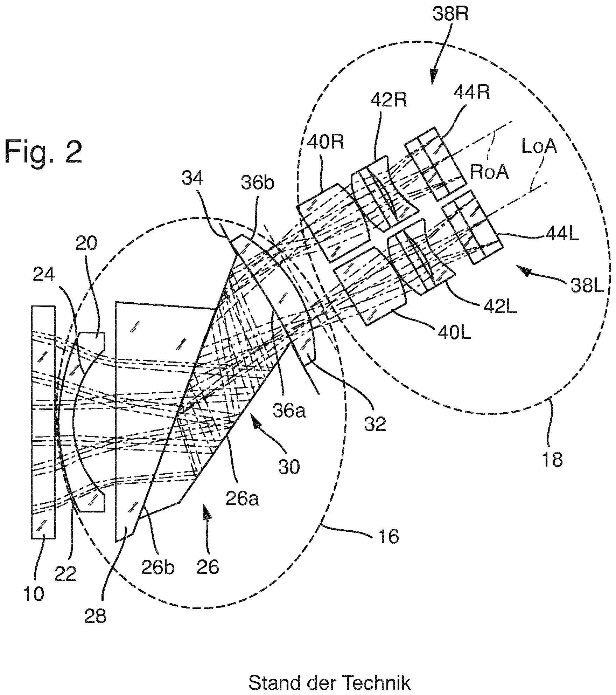

Fig. 2 zeigt in einer vereinfachten schematischen Schnittansicht ein optisches System, wie es beispielsweise aus DE 10 2013 215 422 A1 der Olympus Winter & Ibe GmbH, Hamburg, bekannt ist. Das optische System umfasst eine seitwärtsblickende distale optische Baugruppe 16, welche hinter dem Eintrittsfenster 10 angeordnet ist. Beispielsweise befindet sich das dargestellte optische System im distalen Abschnitt 12 des in Fig. 1 gezeigten Stereo-Videoendoskops 2. Neben der distalen optischen Baugruppe 16 umfasst das optische System eine proximale optische Baugruppe 18. Diese ist beispielsweise durch Drehung des Drehrads 14 im Endoskopschaft 6 drehbar.

Die distale optische Baugruppe 16 umfasst eine Eintrittslinse 20, die beispielsweise als erhabener negativer Meniskus ausgebildet ist. Sie umfasst eine konvexe äußere Oberfläche 22 und eine konkave innere Oberfläche 24. Das von der linken Seite her durch das Sichtfenster 10 eintretende Licht durchquert die Eintrittslinse 20 und fällt in eine als Prismeneinheit ausgebildete Ablenkeinheit 26. Diese umfasst zwei Prismen mit einer teilverspiegelten bzw. verspiegelten Grenzfläche. Das schräg von der Seite her einfallende Licht wird durch die Ablenkeinheit 26 in Richtung einer Längsachse des Endoskopschafts 6 umgelenkt. Die Ablenkeinheit 26 umfasst ein erstes teilverspiegeltes Prisma 28, welches die teilverspiegelte Grenzfläche 26b umfasst. Ferner umfasst die Ablenkeinheit 26 ein weiteres teilverspiegeltes Prisma 30, welches nicht näher dargestellt ist und die verspiegelte Grenzfläche 26a umfasst.

Ferner umfasst die distale optische Baugruppe 16 eine Austrittslinse 32, die in Lichteinfallsrichtung hinter einer Blende 34 angeordnet ist. Alternativ ist zwischen der Austrittslinse 32 und der Ablenkeinheit 26 keine Blende vorgesehen. In diesem Fall sind in Lichteinfallsrichtung vor Stablinsen 40L, 40R der proximalen optischen Baugruppen 18 für einen linken Linsensystemkanal 38L und einen rechten Linsensystemkanal 38R jeweils Blenden angeordnet.

In die Austrittslinse 32 tritt das Licht ausgehend von der Ablenkeinheit 26 ein. Die Austrittslinse 32 ist beispielhaft als hohle positive Meniskuslinse ausgebildet. Sie weist eine konkave Eintrittsfläche 36a und eine konvexe Austrittsfläche 36b auf. Dabei ist der Krümmungsradius der konkaven Eintrittsfläche 36a größer als der Krümmungsradius der konvexen Austrittsfläche 36b.

Nach einer kurzen Strecke erreicht das aus der Austrittslinse 32 austretende Licht die proximale optische Baugruppe 18. Diese umfasst einen linken Linsensystemkanal 38L und einen rechten Linsensystemkanal 38R. Die beiden Linsensystemkanäle 38L, 38R sind gleichartig aufgebaut und zueinander parallel angeordnet. Der linke optische Kanal weist eine linke optische Achse LoA und der rechte optische Kanal weist eine rechte optische Achse RoA auf. Die optischen Achsen LoA, RoA sind zumindest näherungsweise parallel zueinander orientiert. Die beiden Linsensystemkanäle 38L, 38R umfassen jeweils eine Stablinse 40L, 40R, in die das Licht ausgehend von der Austrittslinse 32 der distalen optischen Baugruppe 16 zuerst eintritt. An die linke und rechte Stablinse 40L, 40R schließt sich in Lichteinfallsrichtung jeweils eine Achromaten-Linsengruppe 42L, 42R an. Die Achromaten-Linsengruppen 42L, 42R sind jeweils als Tripletts ausgebildet. Von diesen wird das Licht auf den linken bzw. rechten Bildsensor 44L, 44R gelenkt, so dass der vor der distalen Spitze 8 des Endoskopschafts 6 liegende Untersuchungs- oder Operationsraum stereoskopisch abgebildet wird.

Weitere Einzelheiten zum Aufbau des in Fig. 2 gezeigten optischen Systems sind der genannten DE 10 2013 215 422 A1 zu entnehmen.

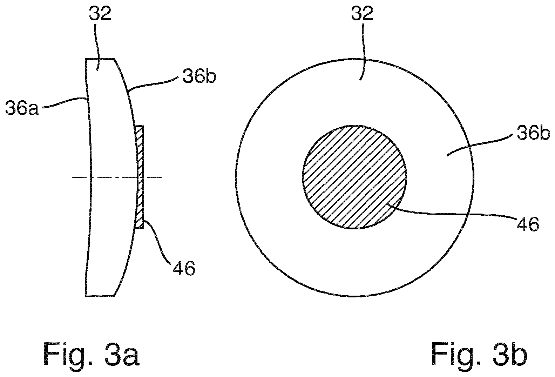

In den Fig. 3a und 3b sind eine Querschnittsansicht (Fig. 3a) und eine Frontansicht (Fig. 3b) einer Austrittslinse 32 der distalen optischen Baueinheit 16 (vgl. Fig. 2) gemäß einer erfindungsgemäßen Ausführungsform schematisch dargestellt. Die Austrittslinse 32 ist hierbei als konkav-konvexe Linse mit einer konkaven Eintrittsfläche 36a und einer konvexen Austrittsfläche 36b ausgebildet.

Erfindungsgemäß ist im mittleren Bereich auf der konvexen Austrittsfläche 36b eine kreisförmige Beschichtung 46 ausgebildet, so dass im Bereich der Beschichtung 46 kein Licht von der Beschichtung 46 im mittleren Bereich hindurchgelassen wird. Insbesondere ist die Beschichtung als kreisförmige Fläche auf der konvexen Austrittsfläche ausgebildet. Vorzugsweise ist dabei die Beschichtung 46 als Chrombeschichtung ausgebildet.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Stereo-Videoendoskop
- 4: Handgriff
- 6: Endoskopschaft
- 8: distale Spitze
- 10: Sichtfenster
- 12: distaler Abschnitt
- 14: Drehrad
- 16: distale optische Baugruppe
- 18: proximale optische Baugruppe
- 20: Eintrittslinse
- 22: äußere Oberfläche
- 24: innere Oberfläche
- 26: Ablenkeinheit
- 26a, 26b: Grenzfläche
- 28: teilverspiegeltes Prisma
- 30: weiteres teilverspiegeltes Prisma
- 32: Austrittslinse
- 34: Blende
- 36a: konkave Eintrittsfläche
- 36b: konvexe Austrittsfläche
- 38L: linker Linsensystemkanal
- 38R: rechter Linsensystemkanal
- 40L, 40R: Stablinse
- 42L, 42R: Achromaten-Linsengruppe
- 44L, 44R: Bildsensor
- 46: Beschichtung

- LoA: linke optische Achse
- RoA: rechte optische Achse

## Patentansprüche

1. Optisches System eines Stereo-Videoendoskops (2) mit seitlicher Blickrichtung, umfassend eine seitwärts blickende distale optische Baugruppe (16) und eine proximale optische Baugruppe (18), wobei die distale optische Baugruppe (16) in einer Lichteinfallsrichtung aufeinanderfolgend eine Eintrittslinse (20), eine als Prismeneinheit ausgebildete Ablenkeinheit (26) und eine Austrittslinse (32) auf einer gemeinsamen optischen Achse umfasst, und wobei die proximale optische Baugruppe (18) einen linken und einen rechten Linsensystemkanal (38L, 38R) umfasst, wobei die Linsensystemkanäle (38L, 38R) gleichartig aufgebaut und parallel zueinander angeordnet sind und jeweils eine eigene optische Achse (LoA, RoA) aufweisen, **dadurch gekennzeichnet, dass** die Austrittslinse (32) im mittleren Bereich der Austrittslinse (32) auf ihrer der Ablenkeinheit (26) abgewandten Lichtaustrittsseite (36b) mit einer lichtundurchlässigen Beschichtung (46) ausgebildet ist.

2. Optisches System nach Anspruch 1, **dadurch gekennzeichnet, dass** die Austrittslinse (32) im mittleren Bereich der Austrittslinse (32) auf ihrer der Ablenkeinheit (26) zugewandten Lichteintrittsseite (36a) mit einer lichtundurchlässigen Beschichtung (46) ausgebildet ist.

3. Optisches System nach Anspruch 1 oder 2, **dadurch gekennzeichnet, dass** die Beschichtung (46) auf der Austrittslinse auf ihrer der Ablenkeinheit (26) abgewandten Lichtaustrittsseite (36b) kreisförmig oder kreisartig ausgebildet ist und/oder dass die Beschichtung (46) auf der Austrittslinse auf ihrer der Ablenkeinheit (26) zugewandten Lichtaustrittsseite (36a) kreisförmig oder kreisartig ausgebildet ist.

4. Optisches System nach einem der Ansprüche 1 bis 3, **dadurch gekennzeichnet, dass** die Austrittslinse (32) für den linken Linsensystemkanal (38L) und den rechten Linsensystemkanal (38R) der proximalen optischen Baugruppe (18) lichtdurchlässig ist.

5. Optisches System nach einem der Ansprüche 1 bis 4, **dadurch gekennzeichnet, dass** die von der Ablenkeinheit (26) abgewandte Seite der Austrittslinse (32) konvex ist.

6. Optisches System nach einem der Ansprüche 1 bis 5, **dadurch gekennzeichnet, dass** die Austrittslinse (32) als konkav-konvexe Linse ausgebildet ist.

7. Optisches System nach einem der Ansprüche 1 bis 6, **dadurch gekennzeichnet, dass** die Beschichtung (46) auf der Austrittslinse (32) auf ihrer der Ablenkeinheit (26) abgewandten Lichtaustrittsseite (36b) als Antireflexbeschichtung (46) ausgebildet ist und/oder dass die Beschichtung (46) auf der Austrittslinse (32) auf ihrer der Ablenkeinheit (26) zugewandten Lichtaustrittsseite (36a) als Antireflexbeschichtung (46) ausgebildet ist.

8. Optisches System nach Anspruch 7, **dadurch gekennzeichnet, dass** die Antireflexbeschichtung (46) als Chrombeschichtung (46) ausgebildet ist.

9. Stereo-Videoendoskop (2) mit fester Blickrichtung mit einem optischen System (20) nach einem der Ansprüche 1 bis 8.

10. Verwendung einer Linse (32) für ein optisches System (20) eines Stereo-Videoendoskops (2) mit fester Blickrichtung nach einem der Ansprüche 1 bis 8, wobei die Linse (32) als Austrittslinse (32) einer distalen optischen Baugruppe (16) ausgebildet ist und im mittleren Bereich der Austrittslinse (32) auf ihrer einer Ablenkeinheit (26) der distalen optischen Baugruppe (16) abgewandten Lichtaustrittsseite (36b) mit einer lichtundurchlässigen Beschichtung (46) ausgebildet ist.

## Claims

1. An optical system of a stereo-video endoscope (2) with a sideways viewing direction, comprising a sideways-viewing distal optical assembly (16) and a proximal optical assembly (18), wherein the distal optical assembly (16) comprises an inlet lens (20), a deflecting unit (26) designed as a prism unit, and an outlet lens (32) on a common optical axis one after the other in the direction of light incidence, and the proximal optical assembly (18) comprises a left and a right lens system channel (38L, 38R), wherein the lens system channels (38L, 38R) are identically designed and are arranged parallel to each other, and each lens system channel has its own optical axis (LoA, RoA), **characterized in that** the outlet lens (32) is formed with a light-impermeable coating (46) on the light outlet side (36b) facing away from the deflecting unit (26) in the central region of the outlet lens (32).

2. The optical system according to claim 1, **characterized in that characterized in that** the outlet lens (32) is formed with a light-impermeable coating (46) on the light inlet side (36a) facing the deflecting unit (26) in the central region of the outlet lens (32).

3. The optical system according to claim 1 or 2, **characterized in that** the coating (46) on the outlet lens is formed in the form of a circle or in the manner of a circle on the light outlet side (36b) facing away from the deflecting unit (26) and/or the coating (46) on the outlet lens is formed in the form of a circle or in the manner of a circle on the light inlet side (36a) facing the deflecting unit (26).

4. The optical system according to any one of claims 1 to 3, **characterized in that** the outlet lens (32) for the left lens system channel (38L) and the right lens system channel (38R) of the proximal optical assembly (18) is permeable to light.

5. The optical system according to any one of claims 1 to 4, **characterized in that** the side of the outlet lens (32) facing away from the deflecting unit (26) is convex.

6. The optical system according to any one of claims 1 to 5, **characterized in that** the outlet lens (32) is formed as a concaveconvex lens.

7. The optical system according to any one of claims 1 to 6, **characterized in that** the coating (46) on the outlet lens (32) is formed as an anti-reflex coating (46) on the light outlet side (36b) facing away from the deflecting unit (26) and/or that the coating (46) on the outlet lens (32) is formed as an anti-reflex coating (46) on the light inlet side (36a) facing the deflecting unit (26).

8. The optical system according to claim 7, **characterized in that** the anti-reflex coating (46) is formed as a chromium coating (46).

9. A stereo-video endoscope (2) having a fixed viewing direction having an optical system (20) according to any one of claims 1 to 8.

10. Use of a lens (32) for an optical system (20) of a stereo-video endoscope (2) having a fixed viewing direction according to any one of claims 1 to 8, wherein the lens (32) is formed as an outlet lens (32) of a distal optical assembly (16) and is formed with a light-impermeable coating (46) on the light outlet side (36b) facing away from a deflecting unit (26) of the distal optical assembly (16) in the central region of the outlet lens (32).

## Revendications

1. Système optique d'un vidéo-endoscope stéréo (2) à vision latérale, comprenant un ensemble optique distal (16) à vision latérale et un ensemble optique proximal (18), l'ensemble optique distal (16) comprenant successivement, dans une direction d'incidence de la lumière, une lentille d'entrée (20), une unité de déviation (26) conçue sous la forme d'une unité formant prisme et une lentille de sortie (32) sur un axe optique commun, et l'ensemble optique proximal (18) comprenant un canal de système de lentilles gauche et un canal de système de lentilles droit (38L, 38R), les canaux de système de lentilles (38L, 38R) étant de construction identique et disposés parallèlement l'un à l'autre et présentant chacun un axe optique propre (LoA, RoA), **caractérisé en ce que** la lentille de sortie (32) est réalisée avec un revêtement opaque (46) dans la zone centrale de la lentille de sortie (32) sur son côté de sortie de lumière (36b) opposé à l'unité de déviation (26).

2. Système optique selon la revendication 1, **caractérisé en ce que** la lentille de sortie (32) est réalisée avec un revêtement opaque (46) dans la zone centrale de la lentille de sortie (32) sur son côté d'entrée de lumière (36a) tourné vers l'unité de déviation (26).

3. Système optique selon la revendication 1 ou la revendication 2, **caractérisé en ce que** le revêtement (46) sur la lentille de sortie sur son côté de sortie de lumière (36b) opposé à l'unité de déviation (26) est de forme circulaire ou de type circulaire, et/ou **en ce que** le revêtement (46) sur la lentille de sortie sur son côté de sortie de lumière (36a) tourné vers l'unité de déviation (26) est de forme circulaire ou de type circulaire.

4. Système optique selon l'une des revendications 1 à 3, **caractérisé en ce que** la lentille de sortie (32) du canal gauche (38L) du système de lentilles et du canal droit (38R) du système de lentilles de l'ensemble optique proximal (18) est translucide.

5. Système optique selon l'une des revendications 1 à 4, **caractérisé en ce que** la face de la lentille de sortie (32) opposée à l'unité de déviation (26) est convexe.

6. Système optique selon l'une des revendications 1 à 5, **caractérisé en ce que** la lentille de sortie (32) est conçue sous la forme d'une lentille concave-convexe.

7. Système optique selon l'une des revendications 1 à 6, **caractérisé en ce que** le revêtement (46) sur la lentille de sortie (32) est réalisé sous forme de revêtement antireflet (46) sur son côté de sortie de lumière (36b) opposé à l'unité de déviation (26), et/ou **en ce que** le revêtement (46) sur la lentille de sortie (32) est réalisé sous forme de revêtement antireflet (46) sur son côté de sortie de lumière (36a) tourné vers l'unité de déviation (26).

8. Système optique selon la revendication 7, **caractérisé en ce que** le revêtement antireflet (46) est réalisé sous forme de revêtement chromé (46).

9. Vidéo-endoscope stéréo (2) à direction de vision fixe, comprenant un système optique (20) selon l'une des revendications 1 à 8.

10. Utilisation d'une lentille (32) pour un système optique (20) d'un vidéo-endoscope stéréo (2) à direction de vision fixe selon l'une des revendications 1 à 8, dans laquelle la lentille (32) est réalisée sous forme de lentille de sortie (32) d'un ensemble optique distal (16) et est réalisée dans la zone centrale de la lentille de sortie (32) sur son côté de sortie de lumière (36b) opposé à une unité de déviation (26) de l'ensemble optique distal (16) avec un revêtement opaque (46).
